(19) Europäisches Patentamt / European Patent Office / Office européen des brevets

(11) **EP 3 373 945 B1**

(12) **EUROPEAN PATENT SPECIFICATION**

(45) Date of publication and mention
of the grant of the patent:
**12.08.2020 Bulletin 2020/33**

(21) Application number: **16808920.9**

(22) Date of filing: **10.11.2016**

(51) Int Cl.:
*A61K 36/28* (2006.01)     *A61P 1/16* (2006.01)

(86) International application number:
**PCT/EP2016/077246**

(87) International publication number:
**WO 2017/081140 (18.05.2017 Gazette 2017/20)**

(54) **AN ORAL DRUG FORMULATION COMPRISING SILYBIN**

ORALE FORMULIERUNG ENTHALTEND SILYBIN

FORMULATION MÉDICAMENTEUSE ORALE COMPRENANT DE LA SILYBINE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **12.11.2015 IT UB20155532**

(43) Date of publication of application:
**19.09.2018 Bulletin 2018/38**

(73) Proprietor: **ISTITUTO BIOCHIMICO ITALIANO
GIOVANNI LORENZINI S.p.A.
04011 Aprilia (IT)**

(72) Inventors:
• **DE IASI, Gianluca
04011 Aprilia (IT)**
• **FEOLA, Monica
00040 Castel Gandolfo (IT)**
• **DI MANZANO, Carlo Maria
00046 Grottaferrata (IT)**

(74) Representative: **Cattaneo, Elisabetta et al
Notarbartolo & Gervasi S.p.A.
Viale Achille Papa, 30
20149 Milano (IT)**

(56) References cited:
**EP-A1- 0 209 037     EP-A1- 0 209 038**

• **DATABASE WPI Week 201113 Thomson
Scientific, London, GB; AN 2011-B21988
XP002756247, & CN 101 926 792 A (TIANJIN
KANGHONG MEDICAL TECHNOLOGY DEV) 29
December 2010 (2010-12-29)**
• **DATABASE WPI Week 201226 Thomson
Scientific, London, GB; AN 2011-P81822
XP002756248, & CN 102 228 430 A (INST CHINESE
MATERIA MEDICA CHINA ACAD T) 2 November
2011 (2011-11-02)**
• **DATABASE WPI Week 201137 Thomson
Scientific, London, GB; AN 2011-D03269
XP002756249, & CN 101 953 794 A (TASLY
PHARM GROUP CO LTD) 26 January 2011
(2011-01-26)**
• **DATABASE WPI Week 201614 Thomson
Scientific, London, GB; AN 2015-64956V
XP002756250, & CN 104 887 630 A (UNIV DALIAN
TECHNOLOGY) 9 September 2015 (2015-09-09)**

**Description**

**FIELD OF THE INVENTION**

The present invention concerns a composition suitable for oral administration comprising silybin.

**STATE OF THE ART**

Silybum marianum Gaertn., commonly known as milk thistle (MT), has been used as a medicinal plant for almost 2 millennia. It is one of the oldest and most extensively studied plants in the treatment of liver diseases. The fruit of milk thistle has been traditionally used for more than 2000 years to treat liver and gallbladder disorders, including hepatitis, cirrhosis, and jaundice, and to protect the liver against poisoning from chemical and environmental toxins, including snake bites, insect stings, mushroom poisoning and alcohol.

The active complex of MT is a lipophilic extract and comprises three flavonolignan isomers, collectively known as silymarin. Silybin, which is a mixture of two diastereoisomers (silybin A and silybin B) in approximately 1:1 proportion, is the most abundant and the most active component of the extract. Over the traditional use of milk thistle, several studies have provided evidences of pharmacological activities of silybin in liver diseases. In liver cells, as well as in other types of cells, silybin acts as antioxidant, modulator of inflammation and fibrogenesis and as indirect and/or direct modulator of some intrahepatic metabolic pathways. However, the bioavailability and therefore the therapeutic efficiency of silybin is limited by its very low water solubility (430 mg/l). In fact, silybin or the mixture of flavonoids of MT (silymarin), have low solubility in water, low systemic bioavailability and poor intestinal absorption. The poor intestinal absorption is likely due to two factors. Firstly, flavonoids are multiplering molecules too large to be absorbed by simple diffusion, so as they are not absorbed actively in the intestinal tract. Secondly, flavonoid molecules typically have poor miscibility with oils and other lipids, thus severely limiting their ability to pass across the lipid-rich outer membranes of the enterocytes of the small intestine.

A known formulation for the systemic administration of silybin is an intravenous preparation, which consists in a powder suitable for solution for infusion. Specifically, this intravenous preparation of the prior art comprises a silybin-C-2',3-dihydrogen succinate sodium salt and resulted to be effective in the treatment of acute liver intoxication by mycotoxins (hepatic intoxication by Amanita phalloides) and in the treatment of infection of treatment-resistant HCV patients. These patients resulted to be treated with up to 3-log reductions in HCV RNA levels by silybin infusion formulation, but they failed to response to oral treatments with common MT extracts.

Even if the therapeutic effect can be seen almost immediately with the intravenous administration, since the injection of the drug occurs directly into the vein and so the drug does not need to be absorbed, this dosage form must be administered by trained personnel and require more time than those administered by other routes. During a 2 hour infusion of a drug containing silybin-C-2',3-dihydrogen succinate sodium salt (named as Legalon SIL), in plasma only the ester silybin in unconjugated form was detectable. The elimination from the blood of the drug is so fast that, three hours after the infusion, only small amounts of conjugates of silibinin C-2',3-bis (hydrogen succinate) disodium salt and ester silybin were detected. The blood test showed therefore a rapid elimination and metabolism of silibinin C-2',3-bis (hydrogen succinate) disodium salt. Therefore, with the administration of drug infusion, to avoid intervals of infusion larger than 3-4 hours, from the end of the last infusion is more appropriate.

Furthermore, the intravenous parental administration requires strict adherence to aseptic procedures, and some pain on injection is inevitable, thus worsening the compliance of the patients.

Oral formulation of silybin or silymarin are known, but they contain a very low amount of silybin.

An oral supplement formulation containing an amount of 47 mg of silybin is known. This formulation is prepared as sachet and contains a high amount of sucrose and maltodextrins. In order to prepare an oral drug based on silybin an increase of the amount of silybin in this formulation was attempted, but the formulation showed a precipitate settled on the bottom with less than 52 mg of silybin.

Furthermore, an oral pharmaceutical formulation, that should be prepared at the moment of the use such as sachet, should have a bioavailability comparable to the bioavailability achieved with intravenous administration in order to perform as a drug.

Therefore, it is still felt the need of a drug comprising silybin, which can also have a better compliance than the known drug composition.

**SUMMARY OF THE INVENTION**

The present inventors studied and provided a new oral formulation comprising a milk thistle extract purified in silybin, thus solving the above stated problem.

Specifically the present inventors achieved this object by an oral pharmaceutical formulation comprising a milk thistle extract of silybin with a purity higher than 80%, phospholipids, sorbitol and carragenin, wherein the weight ratio between the amount of silybin and the amount of phospholipids is in the range of 1:1.50 and 1:2.

The phospholipids of the oral formulation either can be present as a separate ingredient or can be part of the milk thistle extract. In this second embodiment of the invention, the phospholipids of the formulation are present in the final formulation since the milk thistle extract contains silybin and phospholipids blend.

The phospholipids of the invention are preferably phosphatidylcholine.

In a still another aspect the invention relates to an oral pharmaceutical formulation in the form of a powder or a granulate, more preferably as a sachet.

## BRIEF DESCRIPTION OF THE DRAWINGS

The characteristics and advantages of the present invention will be apparent from the detailed description reported below, from the Examples given for illustrative and non-limiting purposes, and from the annexed Figures 1-5, wherein:

Figure 1 shows the demographic data of the healthy subjects of the study of the example 2;
Figure 2 shows mean plasma concentration-time curve of silybin after hydrolysis after 350mg silybin infusion (comparative formulation);
Figure 3 shows mean plasma concentration-time curve of silybin after hydrolysis after 300mg silybin sachets of the invention (oral formulation of the invention);
Figure 4 shows mean plasma concentration-time curve of silybin free after 350mg silybin infusion (comparative formulation);
Figure 5 shows mean plasma concentration-time curve of silybin free after 300mg silybin sachets of the invention (oral formulation of the invention).

## DETAILED DESCRIPTION OF THE INVENTION

The present invention concerns an oral pharmaceutical formulation comprising a milk thistle extract of silybin with a purity higher than 80%, phospholipids, sorbitol and carragenin, wherein the weight ratio between the amount of silybin and the amount of phospholipids is in the range of 1:1.50 and 1:2.

The oral pharmaceutical formulation of the invention comprises a milk thistle extract comprising silybin having a purity higher than 80%, preferably higher than 85%.

The phospholipids of the oral formulation either can be present as a separate ingredient or can be part of the milk thistle extract. In this second embodiment of the invention the phospholipids of the formulation are present in the final oral formulation since the milk thistle extract contains silybin having a purity higher than 80% and phospholipids.

The phospholipids of the invention are preferably phosphatidylcholine.

More preferably the milk thistle extract is an extract available on the market under the trade name Siliphos sold by Indena SPA and comprising silybin and phospholipids.

The final oral pharmaceutical formulation comprises silybin having a purity higher than 80% preferably from 4.5%(circa 100 mg) to 9.5% (circa 200mg) more preferably from 6.8% to 7%, still more preferably 6.9% (150 mg).

The amount of sorbitol is preferably from 60 to 66%, more preferably 65%, with respect to the total amount of the formulation.

The amount of carragenin is preferably 2-3% with respect to the total amount of the formulation

The oral pharmaceutical formulation of the invention comprises also excipients selected from the group consisting of flavour enhancers, sweeteners, gliding agents, pigment agents, flavourings colouring agents.

More preferably, the oral formulation of the invention is in the form of a powder or a granulate, more preferably as a sachet.

In the preferred embodiment of the invention, wherein the composition is formulated as sachet the amount of sorbitol is from 1175 to 1267 mg according to the assay of the drug substance to maintain the final weight of sachet corresponding to 2000 mg.

In comparison with the other oral products based on milk thistle or silymarin, this new formulation contains a very high content in silybin for each unit, from 100 to 200 mg, preferably 150 mg, thus being considered a drug.

In a further aspect therefore the invention concerns an oral pharmaceutical formulation according to anyone of claims 1-9 for use in the treatment of chronic liver damage from alcohol, drugs, toxic substances, metabolic causes and viruses.

Experimental part

Example 1. Preparation of the sachet of the invention

The following ingredients in the amounts reported in Table 1 were used to prepare the oral pharmaceutical formulation of the invention in the form of a sachet.

Table 1

| INGREDIENTS | AMOUNT (mg) |
|---|---|
| Milk thistle extract containing silybin with a purity of 85% | 176.0 mg equivalent to Silybin 150.0 |
| Phospholipids | 261 |
| Sorbitol | 1423 |
| Carrageenans | 45.0 |
| Maltodextrin | 150.0 |
| Sodium Saccharinate | 38.0 |
| Silica colloidal anhydrous | 10.0 |
| Titanium dioxide | 16.4 |
| Orange flavour | 36.0 |
| Tangerine flavour | 27.2 |
| Yellow dye E104 | 0.4 |

The phospholipids were sold as EPIKURON®, and corresponded to a de-oiled phosphatidylcholine enriched powdered soy lecithin.

After checking the amounts, the raw materials were loaded into the mixer after having passing them through the sieving/ginning machine. After disconnecting the loading system, the ingredients were mixed for about 30 minutes

Example 2. Preparation of the sachet of the invention

A batch 001 as standard batch was prepared and contained 100.000 sachets equivalent to 200.0 kg of product.

The following ingredients in the amounts reported in Table 2 were used to prepare the oral pharmaceutical formulation of the invention in the form of a sachet.

Table 2

| Ingredients | AMOUNT (mg) |
|---|---|
| Milk thistle extract containing silybin and phospholipids | 437.0 equivalent to 150.0 of silybin and about 261 of phospholipids |
| Sorbitol | 1423 |
| Carrageenans | 45.0 |
| Maltodextrin | 150.0 |
| Sodium Saccharinate | 38.0 |
| Silica colloidal anhydrous | 10.0 |
| Titanium dioxide | 16.4 |
| Orange flavour | 36.0 |
| Tangerine flavour | 27.2 |
| Yellow dye E104 | 0.4 |

The final product contained 150 mg of silybin and about 261 mg of phospholipids in the form of phosphatidylcholine and the milk thistle extract was that one sold as Siliphos® by Indena SPA.

After checking the amounts, the raw materials were loaded by suction into the mixer after having passing them through the sieving/ginning machine. The sequence for introducing the ingredients was the following: sorbitol (half of the total amount), milk thistle extract (Siliphos), carrageenans, maltodextrin, sodium saccharinate, silica colloidal anhydrous, titanium dioxide, orange flavor, tangerine flavor, yellow dye and the other half of the sorbitol. After disconnecting the loading system, the ingredients were mixed for about 30 minutes.

Example 3. Availability study of the oral formulation of the invention

A study in healthy volunteers was performed to compare the availability and plasma concentrations of silybin after the administration of 2 sachets prepared as in example 2 corresponding to 300 mg of silybin (formulation of the invention) with a comparison formulation of the prior art (comparison formulation). Specifically the comparison formulation was one vial of an intravenous infusion of powder comprising 350 mg of silybin (more specifically silybin-C-2',3-dihydrogen succinate sodium salt).

Details about the study

24 healthy volunteers were enrolled in a single dose bioavailability study. The single dose study was performed to assess the "absolute bioavailability" of the oral formulation of silybin of the invention, when compared to the comparison formulation administered as intravenous infusion (1 vial). The reference product contained a silybin-C-2',3-dihydrogen succinate sodium salt. Each vial contained 350 mg of silybin.

In particular, a two-phase study was designed and 24 volunteers were treated in two different phases: In the first one (Phase 1), 8 healthy adult volunteers, randomly selected from the 24 included volunteers, were treated in fasting condition with the comparison formulation, i.e.1 vial of the infusion drug LEGALON SIL administrated according to the information leaflet, In the second phase. i.e. after 7 days wash-out period, all the 24 healthy adult volunteers (the first 8 of the Phase 1 study plus other 16 volunteers) were treated in fasting condition with 2 sachets of silybin (oral formulation of the invention) in a single dose corresponding to 300 mg of silybin (2x150 mg).

In the Phase 1 blood samples were drawn before the start of the infusion (0h) and then at 0.08h, 0.16h, 0.25h, 0.33h, 0.50h, 0.67h, 1h, 1.33h, 1.67h, 2h, 2.5h, 3h, 4h, 5h, 6h and 8 hours after the start of the infusion. In the Phase 2, blood samples were drawn before administration of the drug (0h) and at 0.33h, 0.67h, 1h, 1.5h, 2h, 2.5h, 3h, 4h, 5h, 6h, 8h, 10h, 12h and 24 hours after the drug administration by oral route.

Silybin is a mixture of two diastereoisomers (silybin A and silybin B) and it undergoes an extensive hepatic metabolism, being rapidly conjugated with sulphate and glucuronic acid in the liver. Accordingly both diastereoisomers of silybin (Silybin A and Silybin B) were determined in plasma as free and total after the hydrolysis with betaglucuronidases and aryl sulfatases through appropriate and validated analytical methods. The analytical study was conducted in an Italian analytical center named R&D Labs; this structure being compliant with the Italian law (ASL certificate no. 557 dated 28/07/2010). The analytical work was conducted in compliance with the analytical protocol and R&D Labs laboratory Standard Operating Procedures and according to the applicable principles of Good Laboratory Practices as requested by the CPMP Guidance on the Investigation of Bioavailability and Bioequivalence (CPMP/EWP/QWP/1401/98 Rev. 1).

The bioavailability of the silybin sachets of the invention versus the intravenous infusion of the comparison formulation was evaluated based on statistics comparisons of relevant pharmacokinetic parameters obtained from data of the plasma concentrations. They included the calculation of the Area under the plasma concentration time curve (AUC) and the maximum concentration in the plasma (Cmax). AUC (the area under the plot of plasma concentration of drug (not logarithm of the concentration) against time after drug administration) was used as a measure of drug exposure. It was derived from drug concentration and time so it gave a measure of how much - how long a drug stayed in a body. Cmax referred to the maximum (or peak) concentration that the drug achieved in the plasma after its administration.

Absolute bioavailability compared the bioavailability of the silybin present in the systemic circulation following oral administration (Phase 2) to the bioavailability of the same drug following intravenous administration (Phase 1). The comparison was dose normalized; consequently, the amount absorbed was corrected by dividing the corresponding dose administered. The absolute bioavailability was the dose-corrected area under curve (AUCs) oral divided by intravenous AUCs. The absolute bioavailability was calculated according to the following formula:

$$F_{abs} = 100 \cdot \frac{AUC_{po} \cdot D_{iv}}{AUC_{iv} \cdot D_{po}}$$

Wherein Fabs = absolute bioavailability; po = per os; iv = intravenous; D = administered dose.

Demographic data of the 24 healthy volunteers

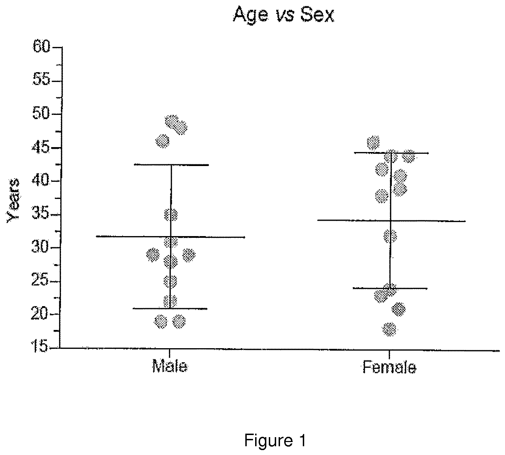

The study was performed on 24 healthy subjects (12 males and 12 females) between 18-55 years of age and BMI between 18.5 and 30 kg/m2. The male and female mean ages were 31.67 and 34.33 years respectively (see figure 1). The youngest was 19 years old (volunteers no. 4 and 5), the oldest was 49 years (volunteer no. 3).

Results of the study

The mean results from the plasma analysis of the volunteers (total N= 24) after the infusion with silybin (8 subjects-PhaseI) and after the treatment with oral sachets (8+16 subjects-Phase 2) were summarized in the following tables and figures .The plasma samples were analyzed for the concentrations of silybin either for the free form or for the total form available after hydrolyses. The plasma concentrations measured after hydrolysis include the products of the liver metabolism of silybin, since silybin was rapidly conjugated with sulphate and glucuronic acid in the liver. The tables 3-6 showed the AUC and Cmax values for Silybin, expressed as the mean value $\pm$ standard deviation (SD).

Table 3. Mean Area under the plasma concentration-time curve (AUC$\pm$SD) of silybin after hydrolysis after 350 mg silybin infusion (Comparative formulation) and 300 mg oral silybin sachets (sachets of the invention).

| $AUC_t$= area under the plasma concentration-time curve = area under the plasma concentration-time curve from zero to time t, where t is the time of the last measurable concentration; | |
|---|---|
| $AUC\infty$ =area under the plasma concentration-time curve from zero to infinity. | |
| $AUC_{0-t}$[(h*ng/ml)]-Mean $\pm$SD | |
| Comparative Intravenous formulation | Formulation of the invention (oral sachets) |
| 4321.80 $\pm$ 2028.14 | 10810.3 $\pm$ 3749.73 |
| $AUC_{0-\infty}$[(h*ng/ml)]-Mean $\pm$SD | |
| 5359.42$\pm$2621.02 | 11212.7 $\pm$3763.64 |

Table 4. Mean Area under the plasma concentration-time curve (AUC) of FREE SILYBIN after 350 mg silybin infusion (Comparative formulation) and 300 mg oral silybin sachets (sachets of the invention).

| $AUC_t$= area under the plasma concentration-time curve = area under the plasma concentration-time curve from zero to time t, where t is the time of the last measurable concentration; | |
|---|---|
| AUC $\infty$ =area under the plasma concentration-time curve from zero to infinity. | |
| $AUC_{0-t}$[(h*ng/ml)]-Mean $\pm$SD | |
| Comparative Intravenous formulation | Formulation of the invention (oral sachets) |
| 453.70$\pm$288.76 | 2877.78$\pm$1242.81 |
| $AUC_{0-\infty}$[(h*ng/ml)]-Mean $\pm$SD | |
| 473.90 $\pm$296.09 | 2954.37 $\pm$1315.71 |

Table 5. Mean peak plasma concentrations of SILYBIN AFTER HYDROLYSIS after 350 mg silybin infusion (Comparative formulation) and 300 mg oral silybin sachets (sachets of the invention).

| Cmax[ng/ml]-Mean $\pm$SD | |
|---|---|
| Comparative Intravenous formulation | Formulation of the invention (oral sachets) |
| 1248.1$\pm$573.50 | 4743.1 $\pm$1824.9 |

Table 6. Mean peak plasma concentrations of free silybin after 350 mg silybin infusion (Comparative formulation) and 300 mg oral silybin sachets (sachets of the invention).

| Cmax[ng/ml]-Mean ±SD | |
|---|---|
| Comparative Intravenous formulation | Formulation of the invention (oral sachets) |
| 206.04 ±129.42 | 2959.3±1442.1 |

As shown in the tables 3-6, the administration of 300 mg of silybin orally (formulation of the invention) allowed to achieve higher peak plasma concentrations and an higher drug exposure during time in comparison to the infusion of silybin, as it can be seen from the Cmax and the AUC mean values. These results were achieved both for free and total silybin (after hydrolysis).

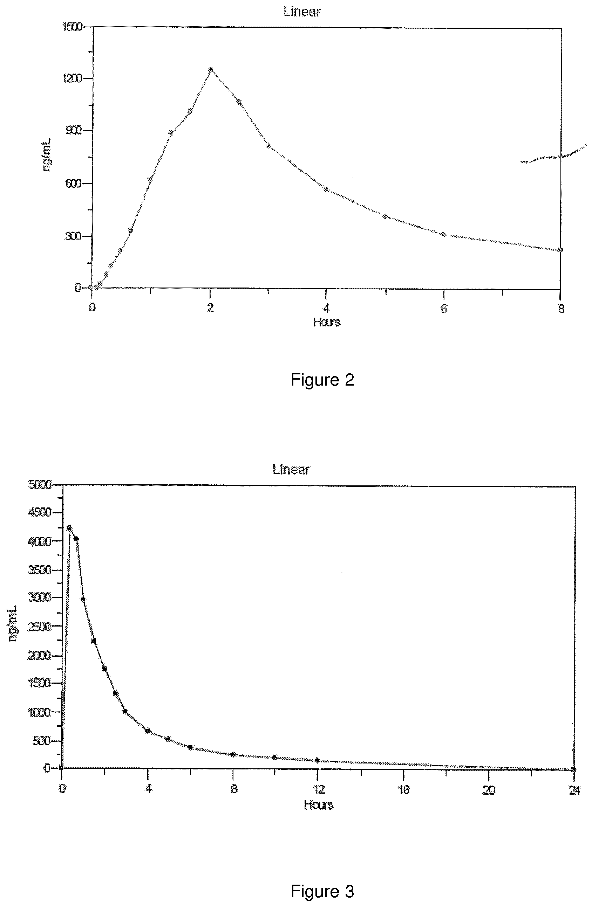

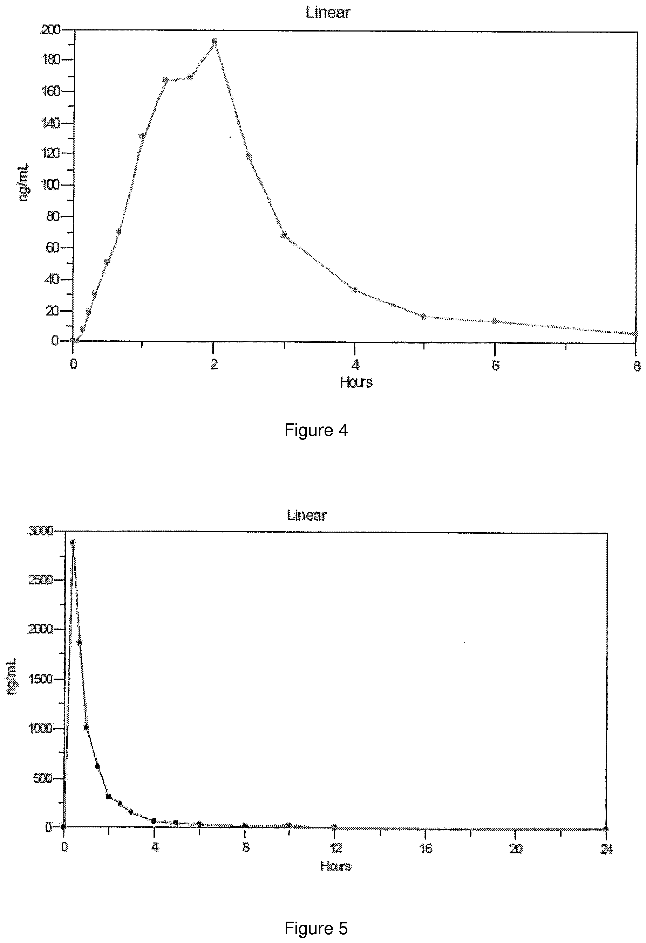

For sake of completeness, the individual plasma levels of silybin measured in 8 volunteers after the infusion with 350 mg of silybin (subj. 3; 6; 10; 12;18;21 ;23) are reported in tables 7-8; the individual plasma levels of silybin measured in all 24 volunteers after the oral administration of 300 mg of silybin (Formulation of the invention) are reported in tables 9-10.The mean values are reported in the figures 2-5.

Table 7. Single plasma levels (ng/mL) of silybin after hydrolysis after 350 mg silybin infusion (Comparative formulation).

| Time (h) | Subj. 3 | Subj. 6 | Subj. 10 | Subj. 12 | Subj. 15 | Subj. 18 | Subj. 21 | Subj. 23 |
|---|---|---|---|---|---|---|---|---|
| 0 | 0.000 | 0.000 | 0.000 | 0000 | 0000 | 0.000 | 0.000 | 0.000 |
| 0.08 | uql | uql | uql | uql | uql | uql | uql | uql |
| 0.16 | uql | 24.413 | uql | uql | uql | 79.893 | 24.288 | 28.047 |
| 0.25 | 28.762 | 82.111 | 32.295 | 87.905 | 75.093 | 136.504 | 80.901 | 72.807 |
| 0.33 | 75.807 | 114.001 | 85.984 | 151.897 | 139.839 | 211.719 | 157.245 | 105.355 |
| 0.50 | 121.721 | 180.602 | 122.435 | 273.357 | 220.460 | 340.165 | 273.053 | 155.385 |
| 0.67 | 133.146 | 244.669 | 186.422 | 493.405 | 341.471 | 460.179 | 539.850 | 201.446 |
| 1 | 221.394 | 594.073 | 463.344 | 1313.256 | 611.972 | 833.000 | 575.571 | 362.515 |
| 1.33 | 554.382 | 670.857 | 611.870 | 1829.524 | 642.653 | 1276.253 | 864,812 | 440.311 |
| 1.67 | 634.014 | 709.201 | 553.712 | 1757.878 | 1105.160 | 1524.109 | 1265.803 | 542.212 |
| 2 | 676.756 | 856.030 | 678.924 | 1981 142 | 1495.359 | 1746.121 | 1845.553 | 705.192 |
| 2.5 | 622.855 | 667.361 | 459.292 | 1618.216 | 1426.515 | 1381.804 | 1715.810 | 585.863 |
| 3 | 523.383 | 416.709 | 347.966 | 1165.604 | 1120.048 | 1099.734 | 1354.384 | 481.776 |
| 4 | 375.737 | 313.763 | 218.101 | 791.072 | 791.488 | 757.337 | 917.297 | 393.099 |
| 5 | 303.901 | 194.598 | 156.926 | 629.694 | 520.074 | 565.620 | 674.292 | 268.275 |
| 6 | 178.840 | 163.812 | 105.108 | 472.934 | 423.144 | 434 388 | 512.967 | 207.779 |
| 8 | 163.229 | 107.726 | 78.238 | 383.127 | 309.078 | 291.178 | 351.826 | 146.203 |
| uql = under quantification limit (for pharmacokinetic calculation it is set as 0) | | | | | | | | |

Table 8. Single plasma levels (ng/mL) of silybin Free after 350 mg silybin infusion (Comparative formulation)

| Time (h) | Subj. 3 | Subj. 6 | Subj. 10 | Subj. 12 | Subj. 15 | Subj. 18 | Subj. 21 | Subj. 23 |
|---|---|---|---|---|---|---|---|---|
| 0 | 0.000 | 0.000 | 0.000 | 0.000 | 0.000 | 0.000 | 0.000 | 0.000 |
| 0.08 | uql | uql | uql | uql | uql | uql | uql | uql |
| 0.16 | 4.909 | 5.616 | 5.328 | 6.964 | 14.055 | 9.527 | 5.831 | 5.388 |
| 0.25 | 7.295 | 15.240 | 14.169 | 27.595 | 26.720 | 21.352 | 16.952 | 14.830 |

(continued)

| Time (h) | Subj. 3 | Subj. 6 | Subj. 10 | Subj. 12 | Subj. 15 | Subj. 18 | Subj. 21 | Subj. 23 |
|---|---|---|---|---|---|---|---|---|
| 0.33 | 14.345 | 22.228 | 18.871 | 43.071 | 45.740 | 40.273 | 33.253 | 23.119 |
| 0.50 | 20.983 | 33.968 | 26.832 | 85.621 | 76.552 | 70.107 | 44.985 | 47.439 |
| 0.67 | 28.556 | 43.369 | 36.603 | 132.019 | 102.353 | 93.586 | 59.219 | 66.388 |
| 1 | 38.694 | 94.585 | 74.556 | 363.934 | 166.269 | 157.253 | 77.686 | 78.535 |
| 1.33 | 71.886 | 96.213 | 96.164 | 434.767 | 228.274 | 211.000 | 107.7134 | 85.718 |
| 1.67 | 94.891 | 82.378 | 73.767 | 328593 | 233.533 | 273.058 | 156.131 | 106.860 |
| 2 | 98.666 | 100.710 | 81.175 | 340.645 | 240.576 | 342.914 | 227.658 | 105.658 |
| 2.5 | 53.217 | 45.202 | 39.333 | 183.133 | 144.529 | 263.292 | 138.942 | 76.499 |
| 3 | 31.871 | 24.232 | 20.271 | 104.742 | 87.905 | 160.801 | 70.612 | 46.268 |
| 4 | 14.704 | 7.703 | 7.077 | 58589 | 54.197 | 71.434 | 25.426 | 23.567 |
| 5 | 7.015 | 4.168 | 3.675 | 38.026 | 25.288 | 35.430 | 11.489 | 11.110 |
| 6 | 4.732 | 3.678 | 2.720 | 46.532 | 12.239 | 21.843 | 6.734 | 7.363 |
| 8 | 5.249 | 3.249 | 2.460 | 14.739 | 5.637 | 11.278 | 2.884 | 4.031 |
| uql = under quantification limit (for pharmacokinetic calculation it is set as 0) | | | | | | | | |

EP 3 373 945 B1

Table 9. Single plasma levels (ng/mL) of silybin after hydrolysis after 300 mg silybin sachets (Formulation of the invention).

| Time (h) | Subj. 1 | Subj. 2 | Subj. 3 | Subj. 4 | Subj. 5 | Subj. 6 |
|---|---|---|---|---|---|---|
| 0 | 0.000 | 0.000 | 0.000 | 0.000 | 0.000 | 0.000 |
| 0.33 | 1360.189 | 3845.347 | 4671.675 | 5104.393 | 3521.134 | 3849.877 |
| 0.67 | 1752.045 | 2980.296 | 5154.510 | 4117.791 | 4512.561 | 4127.459 |
| 1 | 1292.424 | 2264.731 | 3827.329 | 3251.568 | 3405.381 | 2803.507 |
| 1.5 | 851.938 | 1484.133 | 2581.477 | 2322.517 | 3141.101 | 1864.217 |
| 2 | 819.753 | 1156.418 | 2094.312 | 1684.101 | 2026.052 | 1460.196 |
| 2.5 | 949.272 | 1203.019 | 1477.270 | 1303.489 | 1348.958 | 1373.779 |
| 3 | 646.741 | 1066.062 | 1362.335 | 938.498 | 1244.499 | 966.451 |
| 4 | 392.550 | 553.706 | 945.467 | 603.213 | 811.291 | 486.962 |
| 5 | 325.604 | 379.670 | 730.457 | 493.331 | 617.731 | 457.357 |
| 6 | 215.270 | 295.656 | 642.334 | 369.201 | 402.626 | 341.009 |
| 8 | 150.607 | 217.517 | 473.742 | 255.243 | 303.390 | 230.973 |
| 10 | 109.275 | 178.559 | 409.780 | 188.442 | 255.770 | 179.548 |
| 12 | 74.774 | 113.616 | 249.313 | 121.397 | 176.027 | 122.152 |
| 24 | uql | uql | 57.423 | uql | 31.195 | 33.537 |

| Time (h) | Subj. 7 | Subj. 8 | Subj. 9 | Subj. 10 | Subj. 11 | Subj. 12 |
|---|---|---|---|---|---|---|
| 0 | 0.000 | 0.000 | 0.000 | 0.000 | 0.000 | 0.000 |
| 0.33 | 2214.909 | 1615.904 | 2761.070 | 7931.698 | 5562.436 | 5257.760 |
| 0.67 | 4969.641 | 2205.717 | 2630.153 | 5242.480 | 4002.099 | 5666.440 |
| 1 | 4039.809 | 1740.970 | 1818.679 | 4060.975 | 2576.759 | 3033.192 |
| 1.5 | 3270.776 | 1346.596 | 3014.262 | 2658.428 | 2072.928 | 2019.240 |
| 2 | 1973.598 | 799.607 | 2408.238 | 1173.817 | 1791.986 | 1806.712 |
| 2.5 | 1178.183 | 1146.306 | 1485.317 | 868.262 | 1051.908 | 1570.564 |
| 3 | 942.136 | 682.568 | 1259.579 | 856.574 | 812.528 | 993.627 |
| 4 | 666.617 | 479.812 | 873.586 | 445.982 | 847.230 | 879.920 |
| 5 | 626.333 | 325.846 | 704.309 | 364.076 | 450.315 | 761.171 |
| 6 | 458.917 | 256.368 | 483.348 | 248.682 | 360.899 | 512.092 |
| 8 | 233.662 | 152.565 | 336.317 | 190.606 | 360.212 | 352.916 |
| 10 | 175.966 | 98.047 | 267.811 | 195.993 | 176.551 | 350.551 |
| 12 | 113.832 | 90.807 | 150.901 | 89.817 | 137.267 | 246.148 |
| 24 | uql | uql | 39.268 | uql | 24.426 | 35.136 |

Cont'd

uql = under quantification limit (for pharmacokinetic calculation it is set as 0)

9

| Time (h) | Subj. 13 | Subj. 14 | Subj. 15 | Subj. 16 | Subj. 17 | Subj. 18 |
|---|---|---|---|---|---|---|
| 0 | 0.000 | 0.000 | 0.000 | 0.000 | 0.000 | 0.000 |
| 0.33 | 5626.831 | 5430.015 | 369.958 | 5041.007 | 8015.164 | 1036.928 |
| 0.67 | 4206.728 | 3787.594 | 931.738 | 6285.184 | 7351.838 | 1933.906 |
| 1 | 6153.241 | 2072.363 | 614.303 | 4746.652 | 3886.063 | 3538.960 |
| 1.5 | 3622.890 | 2736.559 | 743.582 | 2603.749 | 2490.916 | 3005.775 |
| 2 | 2851.716 | 1742.614 | 1018.103 | 1791.331 | 1616.675 | 3916.983 |
| 2.5 | 2464.555 | 1123.077 | 750.388 | 1509.134 | 1328.138 | 2167.515 |
| 3 | 1806.114 | 674.411 | 783.612 | 1139.685 | 1124.171 | 1499.045 |
| 4 | 854.930 | 421.605 | 416.464 | 737.034 | 664.797 | 1253.344 |
| 5 | 676.097 | 339.980 | 300.928 | 473.132 | 571.301 | 1179.056 |
| 6 | 437.611 | 292.034 | 219.210 | 297.505 | 377.641 | 708.754 |
| 8 | 287.574 | 173.091 | 96.401 | 294.654 | 257.045 | 300.716 |
| 10 | 223.469 | 132.341 | 73.191 | 289.468 | 217.270 | 267.178 |
| 12 | 176.110 | 98.900 | 61.281 | 191.444 | 153.343 | 294.868 |
| 24 | uql | uql | uql | 29.867 | 38.203 | 35.832 |

| Time (h) | Subj. 19 | Subj. 20 | Subj. 21 | Subj. 22 | Subj. 23 | Subj. 24 |
|---|---|---|---|---|---|---|
| 0 | 0.000 | 0.000 | 0.000 | 0.000 | 0.000 | 0.000 |
| 0.33 | 6774.815 | 6015.922 | 5928.080 | 4751.329 | 2271.639 | 2139.298 |
| 0.67 | 6314.753 | 4772.032 | 4348.521 | 3327.109 | 2343.298 | 3355.890 |
| 1 | 3915.242 | 2934.609 | 3168.407 | 2049.846 | 1800.686 | 2197.435 |
| 1.5 | 2415.089 | 2179.442 | 3212.792 | 1073.809 | 1410.216 | 1350.282 |
| 2 | 2200.453 | 2509.375 | 1863.930 | 540.155 | 1505.784 | 1164.809 |
| 2.5 | 1976.379 | 1794.325 | 1376.611 | 537.076 | 872.039 | 869.414 |
| 3 | 1560.202 | 1305.906 | 1229.487 | 352.826 | 659.459 | 250.635 |
| 4 | 1153.881 | 823.350 | 800.625 | 164.394 | 437.579 | 260.771 |
| 5 | 687.175 | 637.362 | 552.322 | 166.610 | 319.395 | 258.926 |
| 6 | 577.673 | 443.629 | 448.741 | 154.809 | 260.487 | 204.747 |
| 8 | 436.218 | 332.032 | 325.637 | 62.126 | 150.058 | 129.007 |
| 10 | 335.996 | 259.324 | 106.932 | 63.412 | 143.560 | 80.002 |
| 12 | 224.860 | 176.746 | 117.065 | uql | 86.676 | 36.847 |
| 24 | 54.304 | 31.431 | uql | uql | uql | uql |

uql = under quantification limit (for pharmacokinetic calculation it is set as 0)

Table 10. Single plasma levels (ng/mL) of silybin free after 300 mg silybin sachets (Formulation of the invention).

| Time (h) | Subj. 1 | Subj. 2 | Subj. 3 | Subj. 4 | Subj. 5 | Subj. 6 |
|---|---|---|---|---|---|---|
| 0 | 0.000 | 0.000 | 0.000 | 0.000 | 0.000 | 0.000 |
| 0.33 | 1069.153 | 2893.610 | 3505.603 | 4428.802 | 3244.023 | 1624.102 |
| 0.67 | 1031.465 | 1590.377 | 2247.119 | 2079.890 | 2707.983 | 1555.698 |
| 1 | 320.283 | 941.573 | 964.812 | 1348.566 | 1461.064 | 522.925 |
| 1.5 | 153.406 | 349.385 | 261.189 | 558.877 | 1220.618 | 259.143 |
| 2 | 155.454 | 204.206 | 158.242 | 271.059 | 399.228 | 275.367 |
| 2.5 | 226.849 | 270.080 | 105.015 | 184.398 | 224.056 | 154.547 |
| 3 | 93.220 | 190.185 | 51.035 | 92.495 | 190.568 | 68.463 |
| 4 | 31.643 | 56.941 | 20.227 | 49.450 | 80.833 | 22.982 |
| 5 | 30.854 | 35.051 | 13.162 | 61.880 | 63.669 | 19.466 |
| 6 | 11.387 | 26.246 | 11.319 | 34.664 | 35.429 | 10.339 |
| 8 | 7.747 | 19.415 | 6.750 | 24.099 | 25.822 | 6.556 |
| 10 | 3.179 | 14.758 | 6.234 | 7.145 | 28.081 | 3.453 |
| 12 | uql | 5.879 | 2.612 | 7.022 | 15.299 | 3.019 |
| 24 | 0.000 | uql | uql | uql | uql | uql |

| Time (h) | Subj. 7 | Subj. 8 | Subj. 9 | Subj. 10 | Subj. 11 | Subj. 12 |
|---|---|---|---|---|---|---|
| 0 | 0.000 | 0.000 | 0.000 | 0.000 | 0.000 | 0.000 |
| 0.33 | 1357.542 | 1020.654 | 1218.024 | 3571.944 | 2722.135 | 3881.624 |
| 0.67 | 1944.370 | 952.180 | 480.140 | 1327.489 | 1500.156 | 2785.854 |
| 1 | 1700.416 | 468.611 | 225.544 | 848.030 | 474.191 | 1058.682 |
| 1.5 | 918.595 | 169.447 | 944.624 | 286.958 | 318.862 | 361.902 |
| 2 | 271.318 | 113.166 | 297.689 | 102.140 | 130.649 | 339.173 |
| 2.5 | 134.454 | 230.218 | 126.140 | 54.468 | 55.448 | 212.802 |
| 3 | 58.050 | 98.923 | 69.600 | 29.319 | 28.770 | 128.557 |
| 4 | 26.540 | 43.911 | 30.730 | 12.376 | 15.077 | 81.279 |
| 5 | 22.409 | 29.299 | 40.200 | 20.542 | 10.695 | 147.511 |
| 6 | 11.731 | 12.003 | 30.905 | 6.379 | 6.696 | 101.878 |
| 8 | 10.528 | 7.003 | 17.974 | 4.002 | 6.846 | 45.862 |
| 10 | 3.990 | 3.805 | 8.026 | 2.696 | uql | 11.185 |
| 12 | uql | 5.973 | 2.615 | uql | uql | 5.003 |
| 24 | 0.000 | 0.000 | 0.000 | uql | 0.000 | uql |

Cont'd

uql = under quantification limit (for pharmacokinetic calculation it is set as 0)

| Time (h) | Subj. 13 | Subj. 14 | Subj. 15 | Subj. 16 | Subj. 17 | Subj. 18 |
|---|---|---|---|---|---|---|
| 0 | 0.000 | 0.000 | 0.000 | 0.000 | 0.000 | 0.000 |
| 0.33 | 3628.110 | 3826.296 | 94.088 | 3769.814 | 6526.776 | 507.341 |
| 0.67 | 1927.010 | 1880.882 | 204.955 | 2268.903 | 3988.619 | 1383.504 |
| 1 | 2315.508 | 909.630 | 89.937 | 1333.288 | 1689.850 | 1197.038 |
| 1.5 | 1744.075 | 847.133 | 56.806 | 509.622 | 712.997 | 1930.914 |
| 2 | 820.805 | 356.328 | 90.462 | 250.424 | 366.526 | 757.482 |
| 2.5 | 811.536 | 168.555 | 78.373 | 152.989 | 249.131 | 686.392 |
| 3 | 376.080 | 81.942 | 117.378 | 88.099 | 182.793 | 375.497 |
| 4 | 80.709 | 35.368 | 33.974 | 37.874 | 70.810 | 138.347 |
| 5 | 61.884 | 34.566 | 13.591 | 39.378 | 44.733 | 77.107 |
| 6 | 34.245 | 12.091 | 5.773 | 30.163 | 26.650 | 50.295 |
| 8 | 11.460 | 7.512 | uql | 13.557 | 16.721 | 26.539 |
| 10 | 8.864 | 5.103 | uql | 15.651 | 13.040 | 11.234 |
| 12 | uql | 2.472 | 0.000 | 16.141 | 10.481 | 12.308 |
| 24 | 13.676 | uql | 0.000 | uql | 0.000 | uql |

| Time (h) | Subj. 19 | Subj. 20 | Subj. 21 | Subj. 22 | Subj. 23 | Subj. 24 |
|---|---|---|---|---|---|---|
| 0 | 0.000 | 0.000 | 0.000 | 0.000 | 0.000 | 0.000 |
| 0.33 | 4956.760 | 3759.718 | 4182.517 | 2591.455 | 1833.081 | 2687.788 |
| 0.67 | 3221.840 | 1850.706 | 1774.851 | 1676.499 | 1677.903 | 2426.306 |
| 1 | 1313.034 | 538.510 | 1157.717 | 787.386 | 1031.528 | 1321.224 |
| 1.5 | 668.809 | 326.702 | 682.012 | 238.512 | 517.664 | 545.110 |
| 2 | 533.739 | 460.308 | 254.933 | 129.659 | 173.926 | 432.190 |
| 2.5 | 499.856 | 323.497 | 115.514 | 84.724 | 113.490 | 262.299 |
| 3 | 296.616 | 147.747 | 59.283 | 67.105 | 403.754 | 161.679 |
| 4 | 218.963 | 48.679 | 22.884 | 32.580 | 60.532 | 40.577 |
| 5 | 117.378 | 29.562 | 10.893 | 22.576 | 37.303 | 36.117 |
| 6 | 79.696 | 14.528 | 11.757 | 8.720 | 19.474 | 31.966 |
| 8 | 56.767 | 6.439 | 4.996 | 5.353 | 9.401 | 16.240 |
| 10 | 46.103 | 3.136 | 3.779 | 3.456 | 5.703 | 6.630 |
| 12 | 20.773 | 5.425 | 2.481 | 2.481 | 4.607 | 5.203 |
| 24 | 9.862 | uql | 0.000 | 3.452 | uql | uql |

uql = under quantification limit (for pharmacokinetic calculation it is set as 0)

The absolute bioavailability was calculated for Silybin either as free or as total after hydrolyses conditions. Due to the fact that the administered formulations showed different dosages in term of amount of silybin (i.e. 350mg for the comparative formulation and 300mg for the formulation of the invention), for the absolute bioavailability (FABS) a correction of the dose was made, and the results are reported in Tables 11 and 12.

Table 11. "Absolute Bioavailability" of silybin after hydrolysis after oral administration of 300 mg of silybin (Formulation of the invention) vs intravenous infusion of 350 mg of silybin (Comparative product).

| Absolute Bioavailability SILYBIN AFTER HYDROLYSIS | | |
|---|---|---|
| | $AUC_{0-t}[(h*ng/ml)]$ | $AUC_{0-\infty}[(h*ng/ml)]$ |
| Comparative formulation | 4321.80 | 5359.42 |
| Formulation of the invention | 10810.3 | 11212.7 |
| FABS | 250 | 209 |
| FABS* | 292 | 244 |
| *= after dose correction (the factor for the dose is 1.167) | | |

Table 12. "Absolute Bioavailability" of free silybin after oral administration of silybin sachets (Formulation of the invention; 300 mg) vs intravenous infusion of the reference formulation (Comparative formulation).

| Absolute Bioavailability SILYBIN FREE | | |
|---|---|---|
| | $AUC_{0-t}[(h*ng/ml)]$ | $AUC_{0-\infty}[(h*ng/ml)]$ |
| Comparative formulation | 453.70 | 473.90 |
| Formulation of the invention | 2877.78 | 2954.37 |
| FABS | 634 | 623 |
| FABS* | 740 | 727 |
| *= after dose correction (the factor for the dose is 1.167) | | |

As shown in the tables 10-11, with reference to the absolute bioavailability, when plasma concentrations of silybin obtained after the oral administration of the formulation of the invention are compared to those obtained after infusion with the comparative formulation, the formulation of the invention showed higher systemic availability of the silybin. More precisely, as far as FABS corrected for the dose is concerned, the systemic availability for silybin given orally with the formulation of the invention was unexpectedly higher than silybin infusion with the comparative formulation, both considering free (more or less 7 times higher) and after hydrolysis (more or less 2 times higher) values.

## Claims

1. An oral pharmaceutical formulation comprising a milk thistle extract of silybin with a purity higher than 80%, phospholipids, sorbitol and carragenin, wherein the weight ratio between the amount of silybin and the amount of phospholipids is in the range of 1:1.50 and 1:2.

2. The oral pharmaceutical formulation according to claim 1 comprises a milk thistle extract comprises silybin having a purity higher than 85%.

3. The oral pharmaceutical formulation according to claim 1 or claim 2 wherein the phospholipids are phosphatidylcholine.

4. The oral pharmaceutical formulation according to anyone of claims 1-3, wherein the milk thistle extract comprises silybin and phospholipids.

5. The oral pharmaceutical formulation according to anyone of claims 1-4, wherein silybin is in the range from 4.5% to 9.5% preferably from 6.8% to 7%, more preferably 6.9% with respect to the total amount of the formulation.

6. The oral pharmaceutical formulation according to anyone of claims 1-5, wherein the amount of sorbitol is from 60 to 66%, preferably 65%, with respect to the total amount of the formulation.

7. The oral pharmaceutical formulation according to anyone of claims 1-6, wherein the amount of carragenin is 2-3% with respect to the total amount of the formulation.

8. The oral pharmaceutical formulation according to anyone of claims 1-7, wherein the oral pharmaceutical formulation comprises also excipients selected from the group consisting of flavour enhancers, sweeteners, gliding agents, pigment agents, flavourings colouring agents.

9. The oral pharmaceutical formulation according to anyone of claims 1-8, wherein the oral formulation is a sachet.

10. An oral pharmaceutical formulation according to anyone of claims 1-9 for use in the treatment of chronic liver damage from alcohol, drugs, toxic substances, metabolic causes and viruses.

**Patentansprüche**

1. Orale pharmazeutische Formulierung, enthaltend einen Mariendistelextrakt von Silybin mit einer Reinheit von über 80 %, Phospholipiden, Sorbitol und Carragenin, wobei das Gewichtsverhältnis zwischen Silybin und der Menge an Phospholipiden im Bereich von 1:1,50 und 1:2 liegt.

2. Orale pharmazeutische Formulierung nach Anspruch 1, die einen Mariendistelextrakt von Silybin mit einer Reinheit von über 85 % enthält.

3. Orale pharmazeutische Formulierung nach Anspruch 1 oder 2, wobei die Phospholipide Phosphatidylcholine sind.

4. Orale pharmazeutische Formulierung nach einem der Ansprüche 1-3, wobei der Mariendistelextrakt Silybin und Phospholipide enthält.

5. Orale pharmazeutische Formulierung nach einem der Ansprüche 1-4, wobei Silybin im Bereich von 4,5 % bis 9,5 %, vorzugsweise von 6,8 % bis 7 %, bevorzugter 6,9 % bezogen auf die Gesamtmenge der Formulierung beträgt.

6. Orale pharmazeutische Formulierung nach einem der Ansprüche 1-5, wobei die Menge an Sorbitol von 60 % bis 66 %, vorzugsweise 65 % bezogen auf die Gesamtmenge der Formulierung beträgt.

7. Orale pharmazeutische Formulierung nach einem der Ansprüche 1-6, wobei die Menge an Carragenin 2-3 % bezogen auf die Gesamtmenge der Formulierung beträgt.

8. Orale pharmazeutische Formulierung nach einem der Ansprüche 1-7, wobei die orale pharmazeutische Formulierung ferner Trägerstoffe umfasst, ausgewählt aus der Gruppe bestehend aus Geschmacksverstärkern, Süßstoffen, Gleitmitteln, Pigmentstoffen, Aroma- und Farbstoffen.

9. Orale pharmazeutische Formulierung nach einem der Ansprüche 1-8, wobei die orale Formulierung ein Beutel ist.

10. Orale pharmazeutische Formulierung nach einem der Ansprüche 1-9 zur Verwendung bei der Behandlung von chronischer Leberschädigung durch Alkohol, Drogen, Giftstoffe, Stoffwechselursachen und Viren.

**Revendications**

1. Une formulation pharmaceutique orale comprenant un extrait de chardon-Marie de silybine avec une pureté supérieure à 80 %, des phospholipides, du sorbitol et du carragénine, dans laquelle le rapport pondéral entre la quantité de silybine et la quantité de phospholipides est dans la plage comprise entre 1/1,50 et 1/2.

2. La formulation pharmaceutique orale selon la revendication 1 comprend un extrait de chardon-Marie comprend de la silybine ayant une pureté supérieure à 85 %.

3. La formulation pharmaceutique orale selon la revendication 1 ou la revendication 2 dans laquelle les phospholipides sont de la phosphatidylcholine.

4. La formulation pharmaceutique orale selon l'une quelconque des revendications 1 à 3, dans laquelle l'extrait de chardon-Marie comprend de la silybine et des phospholipides.

5. La formulation pharmaceutique orale selon l'une quelconque des revendications 1 à 4, dans laquelle la silybine est dans la plage allant de 4,5 % à 9,5 %, préférablement de 6,8 % à 7 %, plus préférablement 6,9 % par rapport à la quantité totale de la formulation.

6. La formulation pharmaceutique orale selon l'une quelconque des revendications 1 à 5, dans laquelle la quantité de sorbitol est de 60 à 66 %, préférablement 65 %, par rapport à la quantité totale de la formulation.

7. La formulation pharmaceutique orale selon l'une quelconque des revendications 1 à 6, dans laquelle la quantité de carragénine est de 2 à 3 % par rapport à la quantité totale de la formulation.

8. La formulation pharmaceutique orale selon l'une quelconque des revendications 1 à 7, dans laquelle la formulation pharmaceutique orale comprend également des excipients sélectionnés dans le groupe constitué par des exhausteurs de goût, des édulcorants, des agents de glissement, des agents de pigmentation, des agents de coloration des arômes.

9. La formulation pharmaceutique orale selon l'une quelconque des revendications 1 à 8, dans laquelle la formulation orale est un sachet.

10. Une formulation pharmaceutique orale selon l'une quelconque des revendications 1 à 9 pour une utilisation dans le traitement d'atteintes hépatiques chroniques dues à l'alcool, aux drogues, aux substances toxiques, aux causes métaboliques et aux virus.

Figure 1

Figure 2

Figure 3

Figure 4

Figure 5